# EUROPEAN PATENT APPLICATION

(11) **EP 2 742 918 A1**
(43) Date of publication of application: **18.06.2014**
(21) Application number: 13197563.3
(22) Date of filing: 16.12.2013
(51) Int. Cl.: A61F 2/95, A61F 2/962, A61F 2/966

(54) **Restraining sheath with variable diameter medical device nesting region**

(30) Priority: 17.12.2012 US 201261738187 P
(71) Applicant: Cook Medical Technologies LLC, Bloomington, IN 47404 (US)
(72) Inventor: Headley, Josh P., Ellettsville, Indiana 47429 (US); Puckett, Dean R., Bloomington, Indiana 47403 (US)
(74) Representative: Garratt, Peter Douglas

(57) **Abstract**

A medical device delivery system is provided including a restraining sheath and a medical device disposed therein. The sheath may include an elongated tubular inner liner having a passageway extending generally longitudinally therethrough. The sheath includes a medical device nesting region located near its distal end. The sheath also may include a strengthening layer and an outer layer. While the outer diameter of the medical device nesting region can be generally constant or partially tapered, an inner diameter of the medical device nesting region generally increases towards the distal end. Methods for manufacturing various components of the medical device delivery system are also disclosed.

## Description

### CROSS-REFERENCE TO RELATED APPLICATIONS

This application claims priority to, and all available benefits of, U.S. Provisional Patent Application No. 61/738,187 filed December 17, 2012.

### BACKGROUND OF THE INVENTION

### 1. Field of the Invention

The disclosure pertains generally to medical devices. More particularly, the disclosure pertains to medical device delivery systems having variable diameter medical device nesting regions.

### 2. Description of the Related Art

Significant advances have been made in a wide variety of minimally invasive medical procedures. Non-limiting examples of such procedures include angioplasty, endoscopy, laparoscopy, and arthroscopy. These minimally invasive procedures can be distinguished from conventional open surgical procedures in that access to a site of concern within a patient is achieved through a relatively small incision, into which a tubular device (or tubular portion of a device) is inserted or introduced. The tubular device, or device portion, keeps the incision open while permitting access to the target site via the interior (i.e., lumen) of the tubular device.

Body passageways in which medical interventional devices are now commonly introduced include the esophagus, trachea, colon, biliary tract, urinary tract, and vascular system, among other locations within the body. One particularly significant example of a minimally invasive technique involves the temporary or permanent implantation of a medical interventional device, such as a stent, into a passageway in the body of a patient.

Stents are useful in a variety of medical procedures and are often used to treat blockages, occlusions, narrowing ailments and other related problems that restrict flow through a passageway. Stents are typically designed as tubular support structures that are implanted within an artery or other vessel at a treatment site and then expanded from a compressed diameter to an expanded diameter. In the expanded state, the stent contacts and radially supports the inner wall of the passageway, thus preventing it from closing. Stents are generally classified as either balloon-expandable or self-expanding. Balloon-expandable stents expand in response to the inflation of a balloon. Self-expanding stents, on the other hand, expand automatically when released from a delivery device.

The delivery device for a self-expanding stent typically includes a catheter or inner carrier about which the stent is mounted in a compressed configuration, and an outer restraining sheath surrounding the stent to maintain it in the compressed configuration for delivery into a body vessel. Once the delivery system is positioned at a treatment site within the vessel, the stent may be deployed to its expanded configuration by retracting the restraining sheath with respect to the inner carrier.

Deployment of medical devices in this manner is now a routine practice. However, when devices such as self-expanding stents are placed in a medical device nesting region of a restraining sheath, a high deployment force may be required due to the outward or radial force exerted by such stents on the interior wall of the nesting region. A higher amount of force will be necessary to withdraw the sheath and overcome the initial stiction forces between the interior surface of the medical device nesting region and the medical device nested therein.

These high deployment forces present numerous challenges. For example, they can cause imprecise placement of a stent at the target site. To address certain challenges, if a relatively long stent length is desired, physicians may decide to use several smaller stents with lower deployment forces and overlap them inside the passageway or conduit at the target site. However, this can lead to undesirable stent migration. Thus, if deployment forces for stents or other medical devices could be reduced, these types of problems could be overcome. Therefore, it is desirable to provide a restraining sheath having a medical device nesting region that lowers deployment forces of medical devices.

### BRIEF SUMMARY OF THE INVENTION

In an aspect of the present disclosure, a medical device delivery system is provided. The medical device delivery system comprises a restraining sheath, a medical device disposed within a distal portion of the restraining sheath, wherein the medical device exerts a radial force on an inner surface of the restraining sheath, and a medical device nesting region within the restraining sheath beginning at a proximal end of the medical device and ending at a distal end of the medical device, wherein an inner diameter of the medical device nesting region increases, and does not decrease, towards a distal end of the medical device nesting region.

Methods for manufacturing various components of the medical device delivery system are also provided by the present disclosure.

### BRIEF DESCRIPTON OF THE SEVERAL VIEWS OF THE DRAWINGS

A detailed description of the invention is hereafter described with specific reference being made to the drawings in which:
Figure 1 shows a partial, cross-sectional view of a tapered embodiment of the restraining sheath and stent nesting region;
Figure 2 shows a partial, cross-sectional view of a stair-step transition embodiment of the restraining sheath and stent nesting region;
Figure 3 shows a partial, cross-sectional view of an alternate stair-step transition embodiment of the restraining sheath and stent nesting region;
Figure 4 shows a partial, cross-sectional view of an alternate tapered embodiment of the restraining sheath and stent nesting region;
Figure 5 shows a partial, cross-sectional view of a tapered and stair-stepped embodiment of the restraining sheath and stent nesting region; and
Figure 6 shows a partial, cross-sectional view of an alternate tapered and stair-stepped embodiment of the restraining sheath and stent nesting region.

### DETAILED DESCRIPTION OF THE INVENTION

As will be more fully described hereinafter, the present disclosure relates to delivery systems capable of reducing the forces necessary to deploy medical devices. Unless defined otherwise, all technical and scientific terms used herein have the same meaning as commonly understood to one of ordinary skill in the art to which this disclosure pertains. In case of conflict, the present document, including definitions, will control. Preferred systems, components, methods, and materials are described below, although systems, methods, components, and materials similar or equivalent to those described herein can be used in the practice or testing of the present disclosure. The systems, components, materials, methods, and examples disclosed herein are illustrative only and not intended to be limiting.

In the present disclosure, the terms "proximal" and "distal" will be used to describe the opposing axial ends of particular components of the medical device delivery system. The term "proximal" is used in its conventional sense to refer to the end of the apparatus (or component thereof) that is closest to the operator or physician during use of the apparatus. The term "distal" is used in its conventional sense to refer to the end of the apparatus (or component thereof) that is initially inserted into the patient, or that is furthest from the operator or physician using the device.

Typically, self-expanding medical devices, such as self-expanding stents, are loaded into a self-expanding medical device nesting region of a restraining sheath having a uniform or constant inner diameter. In the expanded state, the stent will have a diameter larger than the diameter of the nesting region. Thus, the stent must be compressed radially inward in order to fit inside of the nesting region. After loading the stent into the nesting region, the stent constantly tries to expand, thereby exerting radial force against the inner surface of the nesting region. In time, this radial force can cause the stent to stick to or embed itself partially into the inner surface of the nesting region. When a physician subsequently attempts to deploy this stent into, for example, a passage or conduit inside of a patient's body, there is an initial stiction force between the stent and the inner surface of the nesting region that must be overcome. Moreover, as the physician withdraws the sheath, thereby exposing the stent and allowing it to expand and deploy, there are friction forces at work between the stent and the inner surface of the nesting region. The aforementioned forces can be characterized as deployment forces and in certain situations, such as when deploying a heavily compressed self-expanding stent of considerable length, these deployment forces can be quite high causing undesirable challenges for the physician. In certain situations, these challenges can lead to imprecise stent placement, for example.

The present disclosure addresses these and other challenges by providing a medical device delivery system comprising a restraining sheath and a self-expanding medical device disposed therein. The self-expanding medical device can be any self-expanding medical device, such as a self-expanding stent, a filter, a stent graft, or the like. The self-expanding medical device is disposed in a medical device nesting region located at or near a distal end of the restraining sheath. The medical device nesting region can be defined as the portion of the restraining sheath housing or contacting the self-expanding medical device when the self-expanding medical device is loaded therein before deployment. That is, when a self-expanding medical device is loaded into the restraining sheath, the medical device nesting region begins at the proximal end of the self-expanding medical device and ends at the distal end of the self-expanding medical device. In certain aspects, after the self-expanding medical device is loaded into the restraining sheath and before deployment, the entire outer surface, or a substantial portion of the outer surface, of the self-expanding medical device is in contact with the inner surface of the nesting region.

An inner diameter of the medical device nesting region can include a taper, a stair-step, multiple stair-steps, or any combination thereof. In certain aspects, the thickness of the nesting region decreases. This can be a gradual decrease, an abrupt decrease, or a combination thereof. In one aspect, the thickness of one or more layers comprising the nesting region can decrease, either gradually, abruptly, or a combination thereof. For example, the thickness of an inner liner of the nesting region can decrease, thereby increasing the inner diameter of the nesting region. Also, the thickness of an outer layer of the nesting region can decrease, thereby increasing the inner diameter of the nesting region. By decreasing the thickness of the inner liner and/or outer layer as you move distally through the medical device nesting region, the proximal end will be more robust and less prone to stretching from the radial forces of a self-expanding medical device nested therein and the distal end will be less robust and have a greater potential to stretch.

This system can be used for deploying a medical device to a target site within the body of a patient. The restraining sheath and medical device nesting region have a passageway extending generally longitudinally therethrough, and the passageway through the nesting region is configured to receive a self-expanding medical device moveably disposed therein.

In certain aspects, the delivery system can also include an inner catheter configured to slidably extend throughout the passageway of the medical device nesting region. A prosthesis, such as a self-expanding stent, can be disposed within the medical device nesting region and can be supported within the region by the inner catheter.

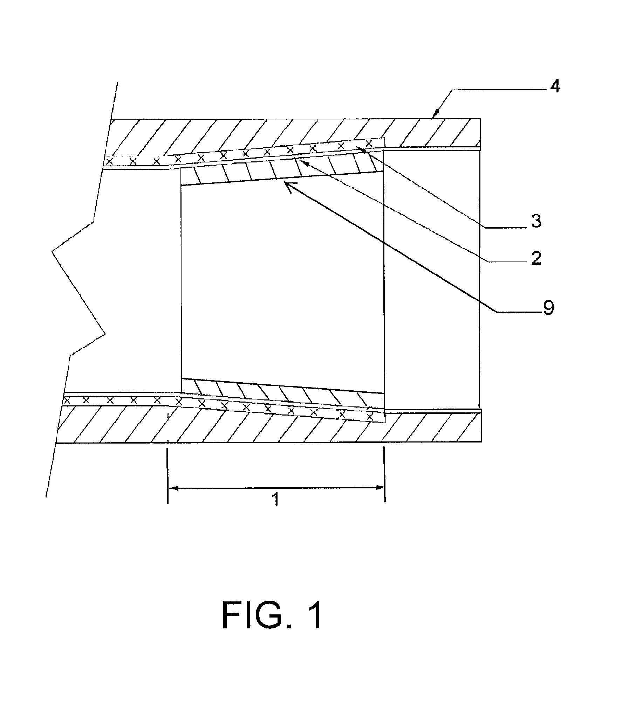

In particular aspects of the present disclosure, such as the aspect depicted in Figure 1, the medical device nesting region (1), which can interchangeably be referred to as a stent nesting region, self-expanding medical device nesting region, self-expanding stent nesting region, or nesting region, may include a cylindrical or tubular shaped inner liner (2), a strengthening layer (3), and an outer layer (4). The outer layer (4) can have a generally constant outer diameter. In certain aspects, the outer diameter of the outer layer (4) can have a slight taper. In particular aspects of this disclosure, the inner diameter of the nesting region (1) is tapered. For example, the inner diameter of the nesting region (1) at the proximal end of the medical device nesting region (1) can have a first diameter and the inner diameter at the distal end of the medical device nesting region (1) can have a second diameter that is larger than the first diameter.

This increase in diameter towards the distal end of the medical device nesting region (1) can be accomplished by tapering the inner surface of the inner liner (2). It can also be accomplished by tapering an inner surface of the outer layer (4). In certain aspects, the taper can be at a gradual slope throughout the nesting region (1) and in other aspects, the angle of slope can vary through the nesting region (1), with the understanding that at all locations within the nesting region (1), the inner diameter of the nesting region (1) either remains constant or increases, but not does not decrease at any point moving towards the distal end. The taper can begin at any location within the nesting region (1) or it can begin at a proximal location to the nesting region (1) and extend into the nesting region (1). Also, the taper can extend to the distal end of the nesting region (1) or it can extend past the distal end of the nesting region (1).

With specific reference to Figure 1, the taper begins near the proximal end of the nesting region (1) and ends near the distal end of the nesting region (1). As can be seen, the outer layer (4) is tapered on its inner surface such that the inner diameter of the nesting region (1) increases substantially throughout the nesting region (1). Moreover, a self-expanding stent (9) is shown nested in the medical device nesting region (1). Again, the portion of the restraining sheath contacting the self-expanding stent (9) before deployment is defined as the medical device nesting region (1).

In all of the drawing figures included herewith, the distal end of the sheath / medical device nesting region is on the right-hand side of the page, from a viewer's perspective, and the proximal end of the sheath / medical device nesting region is on the left-hand side of the page, from a viewer's perspective.

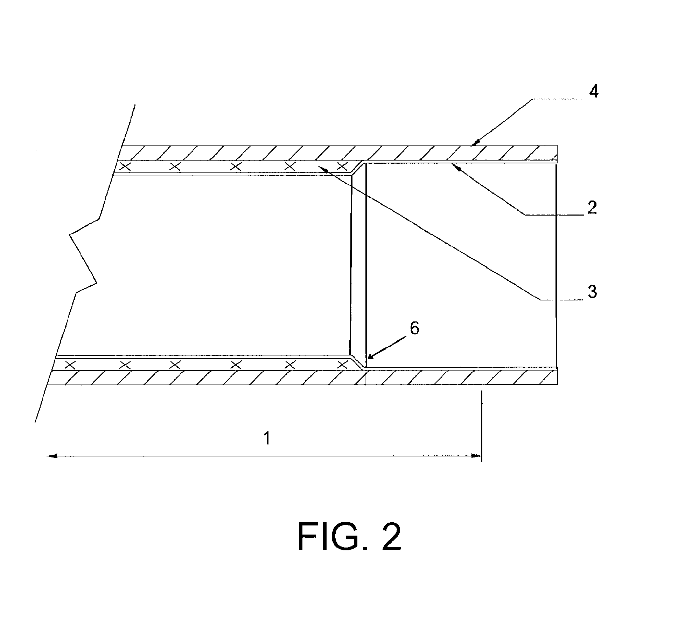

In another aspect, such as in Figure 2, the medical device nesting region (1) can include a stair-step pattern (6) on the inner surface of the inner liner (2). In this aspect, the inner diameter at the proximal end of the medical device nesting region (1) will be smaller than the inner diameter at the distal end of the medical device nesting region (1). Such a stair-step pattern can be accomplished in a variety of ways and certain illustrative, non-limiting examples are set forth hereinafter.

In an aspect of the present disclosure, such as the aspect shown in Figure 2, the medical device nesting region (1) is located near the distal end of the restraining sheath. The nesting region (1) includes an outer layer (4) and an inner liner (2) that extend completely to the distal end of the nesting region (1). A strengthening layer (3) can be disposed in between the outer layer (4) and the inner liner (2). However, in certain aspects, the strengthening layer (3) does not extend completely to the distal end of the medical device nesting region (1). Instead, the strengthening layer (3) stops short of the distal end of the nesting region (1). For example, the strengthening layer (3) can extend from the proximal end of the restraining sheath, into the medical device nesting region (1), and can stop near a mid-point of the nesting region (1). Where the strengthening layer (3) stops, the inner liner (2) will form a stair-step (6) as its inner diameter increases so that its outer surface can come into contact with, and be bonded or mechanically connected to, the inner surface of the outer layer (4). In all aspects, the strengthening layer (3) can stop at any point within the medical device nesting region (1) so that the stair-step (6) is formed at some location within the nesting region (1).

For example, in certain aspects, starting from the proximal end of the medical device nesting region, the outer layer can extend 100% of the length of the nesting region, the strengthening layer can extend from about 5-95% of the length of the nesting region, and the inner liner can extend 100% of the length of the nesting region. In an aspect, starting from the proximal end of the medical device nesting region, the outer layer extends 100% of the length of the nesting region, the strengthening layer extends about 70 - 95% of the length of the nesting region, and the inner liner extends 100% of the length of the nesting region.

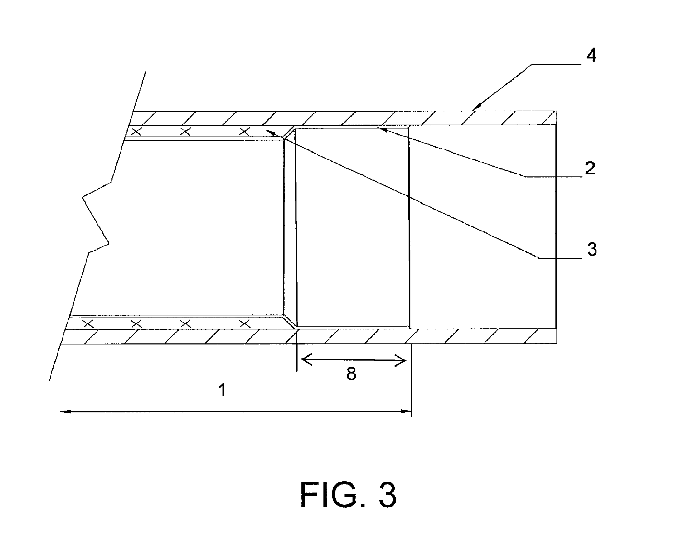

In another aspect, such as the aspect shown in Figure 3, the restraining sheath has a medical device nesting region (1) located near its distal end. The sheath and nesting region (1) include, at their proximal ends, an outer layer (4), a strengthening layer (3), and an inner liner (2). Moving from the proximal end of the nesting region (1) towards the distal end of the nesting region (1), the strengthening layer (3) can stop, while the outer layer (4) and inner liner (2) continue to extend towards the distal end. At the point in the nesting region (1) where the strengthening layer (3) stops, the inner liner (2) will form a stair-step as its inner diameter increases so that its outer surface can come into contact with, and be bonded or mechanically connected to, the inner surface of the outer layer (4). From this first stair-step, the inner liner (2) and outer layer (4) can continue to extend towards the distal end of the medical device nesting region (1). At a distal point to the first stair-step in the nesting region (1), the inner liner (2) can stop while the outer layer (4) continues to extend past the distal end of the nesting region (1) to the distal end of the restraining sheath. Where the inner liner (2) stops, a second stair-step is formed, once again increasing the diameter of the medical device nesting region (1).

In the multiple stair-step embodiments, the strengthening layer (3) can stop at any point within the medical device nesting region (1) so that the first stair-step is formed at some location within the nesting region (1). Also, the inner liner (2) can stop at any point within the medical device nesting region (1), provided that it stops at a distal point from the first stair-step within the nesting region (1). The length of the space in between the first and second stair-steps (8) can vary.

For example, in certain aspects, starting from the proximal end of the medical device nesting region, the outer layer can extend 100% of the length of the nesting region, the strengthening layer can extend from about 5-95% of the length of the nesting region, and the inner liner can extend from about 75-99% of the length of the nesting region, provided that the inner liner extends further towards the distal end of the nesting region than the strengthening layer. In an aspect, starting from the proximal end of the medical device nesting region, the outer layer extends 100% of the length of the nesting region, the strengthening layer extends about 70 - 90% of the length of the nesting region, and the inner liner extends about 91 - 99% of the length of the nesting region.

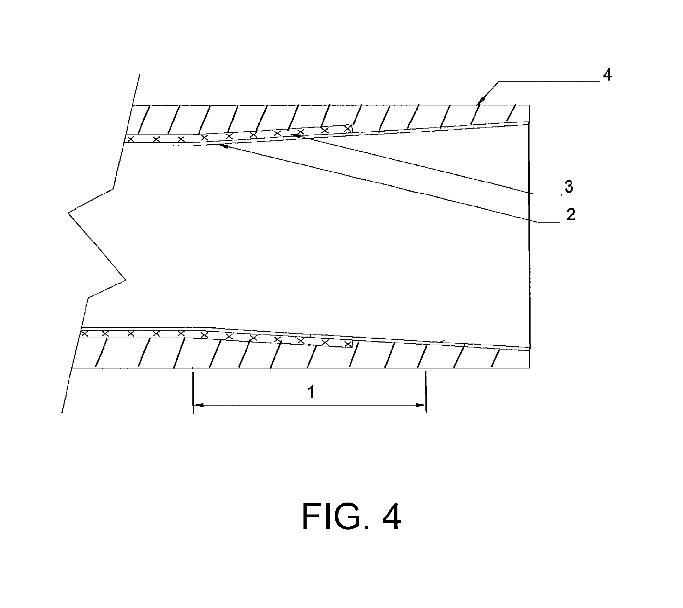

As shown in Figure 4, in certain aspects of the present disclosure, the medical device nesting region (1) is located near the distal end of the restraining sheath. A taper can be introduced starting near the proximal end of the medical device nesting region (1) and the taper can extend past the distal end of the medical device nesting region (1) to the distal end of the restraining sheath. In Figure 4, the inner surface of the outer layer (4) is tapered.

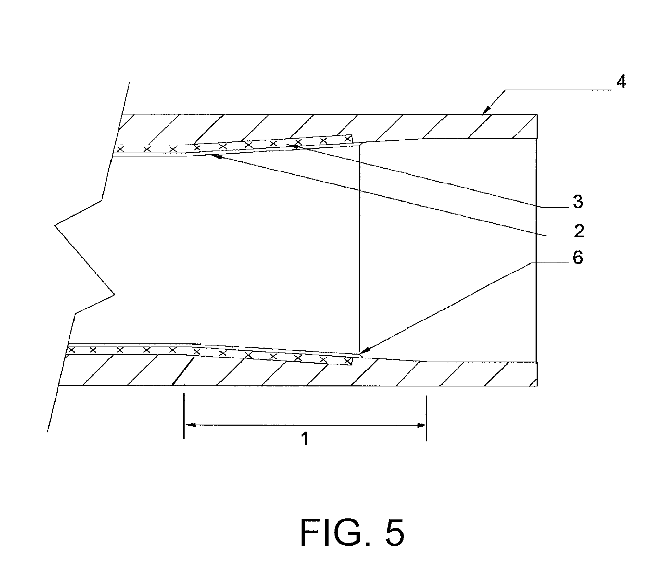

As shown in Figure 5, in certain aspects of the present disclosure, the medical device nesting region (1) can include a taper and a stair-step. The medical device nesting region (1) is located near the distal end of the restraining sheath. The outer layer (4) extends from the proximal end to the distal end of the medical device nesting region (1) and restraining sheath. The strengthening layer (3) extends from a proximal end of the restraining sheath and medical device nesting region (1) but stops short of the distal end of the medical device nesting region (1). In the aspect shown, this does not form a stair-step in the inner diameter of the nesting region (1) because the inner surface of the outer layer (4) is tapered. The inner diameter of the nesting region (1) has a taper starting from the proximal end that extends to a location near the distal end of the nesting region (1). However, the inner liner (2) stops short of the distal end of the medical device nesting region (1), thereby introducing a stair-step (6) into the medical device nesting region (1).

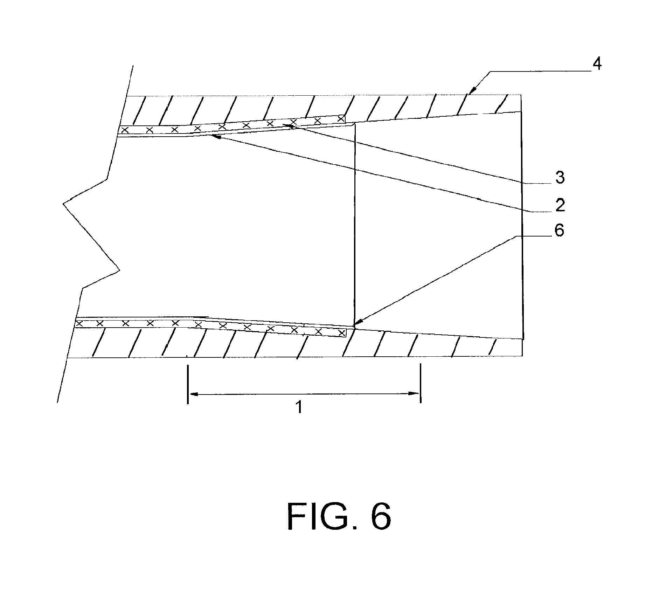

With respect to the embodiment depicted in Figure 6, the restraining sheath has a medical device nesting region (1) located near its distal end. The outer layer (4) has a tapered inner diameter, beginning near the proximal end of the medical device nesting region (1) and extending past the distal end of the nesting region (1) to the distal end of the restraining sheath. The strengthening layer (3) extends from a proximal end of the restraining sheath and medical device nesting region (1) but stops short of the distal end of the medical device nesting region (1). The inner diameter of the inner liner (2) increases starting near the proximal end of the medical device nesting region (1) and continuing distally past the end of the strengthening layer (3). However, the inner liner (2) stops short of the distal end of the medical device nesting region (1), thereby introducing a stair-step (6) into the medical device nesting region (1).

The outer layer, strengthening layer, and inner liner disclosed herein can be formed to any necessary diameters, and such diameters can be selected by one of ordinary skill in the art based upon the intended use of the medical device delivery system. For example, the inner diameter at the proximal end of the medical device nesting region can be from about 1.0 mm to about 1.5 mm and the inner diameter at the distal end of the nesting region can be from about 1.6 mm to about 2.5 mm. In other aspects, the inner diameter at the distal end of the nesting region is from 2% to 10% larger than the inner diameter at the proximal end of the nesting region. It is envisioned that the inner liner can have an inner diameter in the medical device nesting region of up to about 5 mm, or even higher in some instances, at its distal end, with a smaller diameter being found at its proximal end. The wall thickness of the inner liner, as well as the outer layer, can range between about 0.02 mm and about 0.08 mm. Even larger, or smaller, wall thicknesses may be appropriate in a particular case and are covered by the present disclosure.

Also, the length of the restraining sheath and the medical device nesting region can vary and can suitably be selected by one of ordinary skill in the art based upon the intended use of the medical device delivery system. It follows that the longer the medical device being deployed, the longer the medical device nesting region will need to be, such that the medical device can rest in its entirety within the medical device nesting region. In certain aspects, the length of the medical device nesting region can be from about 5 mm to about 250 mm. For example, the medical device nesting region can range from about 50 to about 250 mm, from about 100 to about 250 mm, or from about 150 to about 250 mm. If a medical device longer than 250 mm is being deployed, a longer length medical device nesting region can be selected.

In one aspect, the length of the medical device nesting region is about 220 mm, the inner diameter at its proximal end is about 1.65 mm and the inner diameter at its distal end is about 1.75 mm.

Those skilled in the art will appreciate that all dimensions recited herein are exemplary only, and that the various components of the system described herein may be constructed to be of any size necessary and appropriate to accomplish the purposes for which the system is to be employed.

The outer layer, strengthening layer, and inner liner of the restraining sheath and medical device nesting region can be formed using different types of materials known in the art and the materials can be selected based upon the intended use of the medical device delivery system. In certain aspects, the outer layer comprises a fluoropolymer, such as polytetrafluoroethylene (PTFE). In other aspects, the outer layer comprises a polyamide. For example, the outer layer can comprise a nylon or a similar polymer. In certain aspects, the inner liner comprises a fluoropolymer. For example, the inner liner can comprise PTFE. The inner surface of the inner liner can be lubricious, which will assist when deploying the medical device. In certain aspects, the strengthening layer comprises a metal. The strengthening layer can take the form of a wire having a braided, spiral, or spline configuration. The strengthening layer can comprise a metal such as steel, a metal alloy, a polymer, a metal-polymer composite, ceramics, combinations thereof, or any other suitable material. Inner liners, strengthening layers, and outer layers are well known in the medical arts, and those skilled in the art can readily select an appropriate material for a particular intended use.

In all of the foregoing aspects, a restraining sheath is provided having a medical device nesting region that can lower deployment forces of medical devices, such as stents. As previously noted, while a self-expanding medical device, such as a self-expanding stent, is at rest in a medical device nesting region, the stent exerts a radial force on the inner surface of the nesting region. When loading the stent into the nesting region, it follows that the more the stent must be compressed radially inward in order to fit inside the nesting region, the higher the radial force the stent will exert on the inner surface of the nesting region while the stent is at rest within this region. In the prior art nesting regions, the inner diameter of the nesting region is constant which means that high radial forces are exerted from the proximal end of the stent as well as the distal end of the stent.

With this in mind, one may attempt to simply increase the outer and inner diameters of the entire medical device nesting region, which would reduce the amount of radial force exerted on the inner surface of the nesting region by the stent. However, there are limits to the outer diameter length since eventually this nesting region may need to be inserted into a passageway or conduit within a patient's body. Thus, one may consider increasing the inner diameter of the nesting region, while holding the outer diameter constant, but as the inner diameter increases, the wall of the nesting region will become thinner, thereby leading to a nesting region that becomes very elastic and much less robust. When withdrawing this type of nesting region over the self-expanding medical device, the nesting region will stretch to an undesirable degree, making it very difficult to deploy the medical device. A balance between an increased inner diameter and robustness of the nesting region must be achieved.

Thus, with regard to the present disclosure, it has been discovered that a sufficiently robust medical device nesting region can be created that reduces the radial forces exerted by the self-expanding medical device, which is nested therein. Specifically, the inner diameter of the presently disclosed medical device nesting region increases towards its distal end while the outer diameter of the nesting region remains generally constant or has a slight taper. At a proximal end of the nesting region, the inner diameter will be smaller, thereby compressing the self-expanding medical device to a higher degree than at the distal end, where the inner diameter is larger. However, the thickness of the nesting region wall will be greatest at the proximal end of the nesting region, thereby providing the necessary robustness to the nesting region to handle the higher radial forces and reduce stretching. Within the nesting region, the increase in diameter can be gradual, a stair-step increase, a combination including stair-step and gradual, or, in certain instances, a portion of the inner diameter of the nesting region may be held constant from one point to another. However, the inner diameter of the medical device nesting region does not decrease at any point within the nesting region towards the distal end. With such a design, as you move distally along the stent within the nesting region, the radial forces exerted by the stent on the inner surface of the nesting region will decrease as the inner diameter increases. This allows for reduced deployment forces when compared to prior art nesting regions having constant inner diameters or prior art nesting regions having inner diameters that decrease at certain points as you move distally through the nesting region, such as an inner diameters having grooves or wave-like patterns as disclosed in Figure 3 of US 2010/0145429.

When a physician begins to withdraw the presently disclosed restraining sheath / medical device nesting region to expose and deploy the stent, the distal end of the stent will be exerting a reduced amount of radial force on the inner surface of the nesting region, since the distal end of the nesting region has an increased inner diameter. Since the radial forces are reduced at the distal end of the nesting region, the wall of the nesting region at the distal end can be thinner. Thus, the total deployment force necessary to withdraw the nesting region over the stent will be lowered when compared to a prior art nesting region that does not include an increase in diameter in the nesting region.

Even further, in certain aspects, the medical device nesting region will have an inner profile that is substantially funnel-shaped, with the wider portion of the funnel being located at the distal end of the nesting region. Again, this shape will reduce the radial force being exerted by the stent as you move distally along the stent body and the nesting region, thus lowering total deployment forces. Also, because the stent itself will conform to the funnel-shaped pattern, it will have an inherent force tending to push it out of the distal end of the nesting region, which will also help to lower the force necessary to deploy the stent.

Methods of manufacturing the medical device delivery systems, restraining sheaths, and medical device nesting regions thereof are also disclosed.

Restraining sheaths are well-known in the art, as are methods for manufacturing such sheaths. With respect to manufacturing the medical device nesting region of the presently disclosed restraining sheath, various procedures are contemplated.

In one aspect, a mandrel is provided having a proximal end and a distal end. The outer diameter of the distal end is larger than the outer diameter of the proximal end. The increase in diameter can be due to a gradually sloping taper on the mandrel, a non-uniform taper on the mandrel, one or more stair-step patterns worked into the mandrel, or any combination thereof. An inner liner can be placed over the mandrel and stretched to conform to the shape of the mandrel.

Next, a strengthening layer can be disposed along the outer surface of the inner liner. The strengthening layer would cover a first portion of the outer surface of the inner liner, while leaving a second portion of the outer surface of the inner liner uncovered. In certain aspects, this can be accomplished by winding a wire around the outer surface of the inner liner. The portions of the outer surface that contact the wire are referred to as "first portion" above and those portions of the outer surface of the inner liner that do not contact the wire are referred to as "second portion" above.

An outer layer can then be disposed on the strengthening layer, followed by the application of a final layer of heat shrinkable material over the outer layer. Heat is then applied to the heat shrinkable material of a temperature sufficient to allow the outer layer to bond the second portion of the outer surface of the inner liner. Finally, the final layer of heat shrinkable material is removed, followed by removal of the mandrel.

In accordance with certain aspects of the medical device nesting region disclosed herein, the inner liner, strengthening layer, and outer layer can extend the entire distance of the mandrel or one or more of these layer may not extend the entire length of the mandrel.

For example, in one aspect, starting from the proximal end of the mandrel, the outer layer extends 100% of the length of the mandrel, the strengthening layer extends about 70 - 95% of the length of mandrel, and the inner liner extends 100% of the length of the mandrel.

All of the devices, systems, components, and methods disclosed and claimed herein can be made and executed without undue experimentation in light of the present disclosure. While the disclosed invention may be embodied in many different forms, there are described in detail herein specific embodiments of the invention. The present disclosure is an exemplification of the principles of the invention and is not intended to limit the invention to the particular embodiments illustrated. In addition, unless expressly stated to the contrary, use of the term "a" is intended to include "at least one" or "one or more." For example, "a device" is intended to include "at least one device" or "one or more devices."

Any ranges given either in absolute terms or in approximate terms are intended to encompass both, and any definitions used herein are intended to be clarifying and not limiting. Notwithstanding that the numerical ranges and parameters setting forth the broad scope of the invention are approximations, the numerical values set forth in any specific examples are reported as precisely as possible. Any numerical value, however, inherently contains certain errors necessarily resulting from the standard deviation found in their respective testing measurements. Moreover, all ranges disclosed herein are to be understood to encompass any and all subranges (including all fractional and whole values) subsumed therein.

Furthermore, the invention encompasses any and all possible combinations of some or all of the various embodiments described herein. It should also be understood that various changes and modifications to the presently preferred embodiments described herein will be apparent to those skilled in the art. Such changes and modifications can be made without departing from the spirit and scope of the invention and without diminishing its intended advantages. It is therefore intended that such changes and modifications be covered by the appended claims.

## Claims

1. A medical device delivery system comprising:
a restraining sheath;
a medical device disposed within a distal portion of the restraining sheath, wherein the medical device exerts a radial force on an inner surface of the restraining sheath; and
a medical device nesting region within the restraining sheath beginning at a proximal end of the medical device and ending at a distal end of the medical device, wherein an inner diameter of the medical device nesting region increases, and does not decrease, towards a distal end of the medical device nesting region.

2. The medical device delivery system of Claim 1, wherein the restraining sheath comprises a member selected from the group consisting of a strengthening layer, an outer layer, and any combination thereof.

3. The medical device delivery system of Claim 1, wherein the restraining sheath comprises:
an inner liner having a proximal portion, a distal portion, an outer surface, an inner surface, and a passageway extending generally longitudinally therethrough;
a strengthening layer positioned longitudinally around the outer surface of the inner liner; and
an outer layer positioned longitudinally around the strengthening layer.

4. The medical device delivery system of any one of the preceding claims, wherein the inner surface of the restraining sheath is tapered substantially throughout the medical device nesting region toward the distal end of the medical device nesting region.

5. The medical device delivery system of Claim 3, wherein the inner liner extends circumferentially to the distal end of the medical device nesting region, the outer layer extends circumferentially to the distal end of the medical device nesting region, and the strengthening layer extends circumferentially around a portion of the medical device nesting region but stops before the distal end of the medical device nesting region, thereby forming a stair-step in an inner diameter of the inner liner.

6. The medical device delivery system of Claim 3 or Claim 5, wherein the outer layer extends 100% of a length of the medical device nesting region, the strengthening layer extends from about 5% to about 95% of the length of the medical device nesting region, and the inner liner extends 100% of the length of the medical device nesting region.

7. The medical device delivery system of Claim 3, wherein the outer layer extends circumferentially to the distal end of the medical device nesting region, the inner liner extends circumferentially around a portion of the medical device nesting region but stops before the distal end of the medical device nesting region, and the strengthening layer extends circumferentially around a portion of the medical device nesting region but stops before a location where the inner liner ends, thereby forming multiple stair-steps in the medical device nesting region.

8. The medical device delivery system of Claim 3 or Claim 7, wherein the outer layer extends 100% of a length of the medical device nesting region, the strengthening layer extends from about 5% to about 95% of the length of the medical device nesting region, and the inner liner extends from about 75% to about 99% of the length of the medical device nesting region, provided that the inner liner extends further towards the distal end of the medical device nesting region than the strengthening layer.

9. The medical device delivery system of any one of the preceding claims, wherein an inner diameter at a proximal end of the medical device nesting region is from about 1.0 mm to about 1.5 mm and an inner diameter at the distal end of the medical device nesting region is from about 1.6 mm to about 2.5 mm.

10. The medical device delivery system of any one of Claim 3 to Claim 8, wherein the inner liner comprises a fluoropolymer, the strengthening layer comprises a metal, and the outer layer comprises a polyamide or fluoropolymer.

11. The medical device delivery system of any one of the preceding claims, wherein the restraining sheath has a generally constant outer diameter or a partially tapered outer diameter.

12. The medical device delivery system of any one of the preceding claims, wherein the medical device is a self-expanding stent.

13. The medical device delivery system of any one of the preceding claims, wherein an inner diameter at the distal end of the medical device nesting region is from about 2% to about 10% larger than an inner diameter at a proximal end of the medical device nesting region.

14. The medical device delivery system of Claim 3, wherein the inner surface of the outer layer is tapered substantially throughout the medical device nesting region, and further wherein an inner diameter at a proximal end of the medical device nesting region is from about 1.0 mm to about 1.5 mm, an inner diameter at the distal end of the medical device nesting region is from about 1.6 mm to about 2.5 mm, and the medical device nesting region is from about 100 mm to about 250 mm in length.

15. The medical device delivery system of Claim 3, wherein the outer layer extends 100% of a length of the medical device nesting region, the strengthening layer extends from about 5% to about 90% of the length of the medical device nesting region, and the inner liner extends from about 91 % to about 100% of the length of the medical device nesting region, and further wherein an inner diameter at a proximal end of the medical device nesting region is from about 1.0 mm to about 1.5 mm, an inner diameter at the distal end of the medical device nesting region is from about 1.6 mm to about 2.5 mm, and the medical device nesting region is from about 100 mm to about 250 mm in length.
